(19) Europäisches Patentamt
European Patent Office
Office européen des brevets

(11) Publication number: 0 003 415
A2

(12) # EUROPEAN PATENT APPLICATION

(21) Application number: 79300096.9

(22) Date of filing: 19.01.79

(51) Int. Cl.[2]: C 07 D 499/00
C 07 D 205/08, C 07 D 498/04
A 61 K 31/43
//(C07D499/00, 277/00, 205/00),
(C07D498/04, 263/00, 205/00)

(30) Priority: 20.01.78 GB 246478
20.09.78 GB 3752178

(43) Date of publication of application:
08.08.79 Bulletin 79/16

(84) Designated contracting states:
BE CH DE FR GB IT NL SE

(71) Applicant: GLAXO GROUP LIMITED
Clarges House 6-12 Clarges Street
London W1Y 8DH(GB)

(72) Inventor: Watson, Nigel Stephen
5 Ruislip Close
Greenford Middlesex(GB)

(74) Representative: Holmes, Michael John et al,
Frank B. Dehn & Co. Imperial House 15-19 Kingsway
London WC2B 6UZ(GB)

(54) **Beta-Lactam compounds, processes for their preparation, compositions containing them and intermediates of use in their preparation.**

(57) β-Lactam compounds, process for their preparation, compositions containing them and intermediates of use in their preparation are described. The β-lactam compounds have the formula

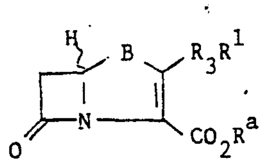

(wherein $R^{2a}$ represents a carboxyl esterifying group or a hydrogen atom, $R_3$ represents a group $-C_nH_{2n}-$ where n is an integer from 1 to 6, $R^1$ represents an azido group $-N_3$ or an amino group $-NH_2$ and B represents >S or >S→O) together with salts and zwitterionic forms thereof.

The acids, salts and metabolically labile esters are of use as antibiotics or as β-lactamase inhibitors against a range of gram-positive and gram-negative microorganisms.

EP 0 003 415 A2

"β-Lactam compounds, processes for their preparation, compositions containing them and intermediates of use in their preparation"

This invention relates to novel antibiotic compounds, processes for their preparation, compositions containing them and intermediates of use in their preparation.

We have found that a new class of 6-unsubstituted penems, more particularly described below, possess useful antibiotic activity, particularly against certain penicillin-resistant microorganisms, or they may be useful as intermediates in the preparation of further active compounds.

According to the present invention we provide compounds of formula (I)

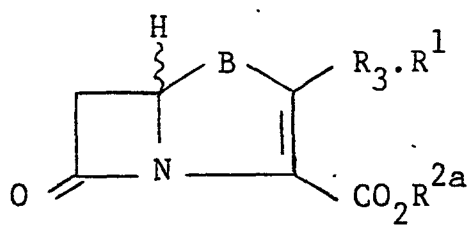

(I)

(wherein $R^{2a}$ represents a carboxyl esterifying group or a hydrogen atom, $R_3$ represents a group $-C_nH_{2n}-$ where n is an integer from 1 to 6, $R^1$ represents an azido group $-N_3$ or an amino group $-NH_2$ and B represents $>S$ or $>S\rightarrow O$) together with salts and zwitterionic forms thereof.

B preferably represents >S.

The group $-C_nH_{2n}-$ represented by $R^3$ may be straight or branched and n is preferably from 1 to 4 as, for example, in a methylene, ethylene or propylene group. Most preferably, $R^3$ is ethylene.

The compounds of formula (I) may exist as individual 5-position isomers or as mixtures thereof and the invention extends to the isomers of the compounds individually or in admixture.

The invention also includes non-toxic physiological precursors of the active compounds of the invention, that is substances which on administration are converted _in vivo_ into the antibiotic compounds. In the case of the acids of formula (I) ($R^{2a}$=H), their metabolically labile esters (which already fall within the definition of the compounds of the invention) are examples of such physiological precursors.

Where $R^{2a}$ represents a hydrogen atom and $R^1$ represents an amino group, the compounds of formula (I) may exist as zwitterions or may form acid addition salts or salts with bases Esters of the compounds in which $R^1$ is an amino group may form acid addition salts and compounds of formula (I) in which $R^1$ is an azido group and $R^{2a}$ is a hydrogen atom may form salts with bases. The salts with bases may be salts with inorganic bases such as alkali metal salts, e.g. sodium, potassium and lithium salts; alkaline earth metal salts, e.g. calcium

and magnesium salts and ammonium salts; or salts with organic bases, for example amine salts. Acid addition salts according to the invention include salts with inorganic acids, e.g hydrochloric, hydrobromic, perchloric, sulphuric or phosphoric acid salts and salts with organic acids e.g. acetic, propionic, citric, maleic, trifluoroacetic or p-toluenesulphonic acid salts.

When $R^{2a}$ represents a carboxyl esterifying group, it may, for example, be an organic group derived from an alcohol (aliphatic or araliphatic), a phenol, a silanol or a stannanol. Such an alcohol, phenol, silanol or stannanol used to esterify the carboxyl group preferably contains not more than 24 carbon atoms.

Thus, the group $R^{2a}$ may represent a straight or branched unsubstituted or substituted alkyl or alkenyl group, preferably having from 1-8 carbon atoms, for example a methyl, ethyl, propyl or isopropyl, butyl, sec-butyl, tert-butyl or allyl group, optional substituents being for example, alkoxy e.g. methoxy; halogen i.e. fluorine, chlorine, bromine or iodine; cyano; acyloxy, e.g. alkanoyloxy, such as acetoxy or pivaloyloxy, or alkoxycarbonyloxy, such as ethoxycarbonyloxy; acyl e.g. p-bromobenzoyl and alkoxycarbonyl e.g. ethoxycarbonyl;

an aralkyl group having up to 20 carbon atoms especially an arylmethyl group e.g. a benzyl or substituted benzyl group, suitable substituents being either halo e.g. chloro; nitro, e.g. o or p-nitro; cyano; alkoxy e.g. p-methoxy or alkyl e.g. p-methyl groups; a diphenylmethyl or triphenylmethyl group or a fur-2-ylmethyl, thien-2-ylmethyl or pyrid-4-ylmethyl group, the heterocyclic groups of which may also be substituted e.g. by a $C_{1-4}$ alkyl group, preferably methyl;

an aryl group having up to 12 carbon atoms e.g. a phenyl or substituted phenyl group, suitable substituents being either halo e.g. chloro; nitro, e.g. o or p-nitro; cyano; alkoxy e.g. p-methoxy or alkyl e.g. p-methyl groups;

a cycloalkyl group containing not more than 12 carbon atoms e.g. adamantyl, cyclohexyl or cyclopentyl;

a heterocyclic group containing not more than 12 carbon atoms, the hetero atom being, for example, oxygen, as in the tetrahydropyranyl or phthalidyl group;

a stannyl group having up to 24 carbon atoms for example a stannyl group carrying three substituents which may be the same or different selected from alkyl, alkenyl, aryl, aralkyl, cycloalkyl, alkoxy, or aralkoxy groups, including methyl, ethyl, propyl, n-butyl, phenyl and benzyl groups;

or a silyl group having up to 24 carbon atoms which may carry three groups, which may be the same or different, selected from alkyl, alkenyl, cycloalkyl, aralkyl and aryl groups, preferably $C_{1-4}$ alkyl groups e.g. methyl, ethyl, n-propyl, iso-propyl or t-butyl groups.

The alkyl, alkoxy, alkanoyloxy, alkanoyl and alkoxycarbonyl substituents referred to above preferably contain 1-4 carbon atoms.

In general, the acids, salts and metabolically labile esters of the compounds of formula (I) in which B represents >S

and salts of such compounds are preferred forms for use in medicine. However, the other esters are also useful and, for example, where the ester grouping is readily cleaved e.g. by hydrolysis or hydrogenolysis, without significant degradation of the rest of the molecule, the esters are especially useful as carboxyl-protected derivatives of the parent compounds. Those esters which are readily cleaved and are primarily of use in this connection include arylmethyl esters, especially the benzyl, o-nitrobenzyl, p-nitrobenzyl, benzhydryl and trityl esters as well as the stannyl, e.g. tri-n-butyl-stannyl esters. Of particular value in this regard are the readily cleaved esters (e.g. the arylmethyl esters, especially the p-nitrobenzyl ester) of the azide compounds, since reduction of these affords the carboxylic acid of the amino compound.

As indicated above, the ester grouping may be metabolically labile, generating the parent acid *in vivo*. Metabolically labile esters include substituted alkyl esters carrying an oxygen substituent on the α-carbon atom, for example, α-acyloxyalkyl esters, e.g. acetoxymethyl and pivaloyloxymethyl esters; α-alkoxycarbonyloxyalkyl esters, e.g. 1-ethoxycarbonyloxyethyl esters; and phthalidyl esters.

The acids, salts and metabolically labile esters of the invention wherein B represents >S possess antibiotic activity. They have been found to be active against a range of gram-positive and gram-negative microorganisms, for example against strains of *Staphylococcus aureus*, *Micrococcus* species, *Escherichia coli*, *Salmonella typhimurium*,

Shigella sonnei, Enterobacter cloacae, Klebsiella aerogenes, Proteus mirabilis, Serratia marcescens, Providence species and Haemophilus influenzae.

In general, the compounds are stable to the action of β-lactamases produced by some gram-positive organisms, for example those produced by Staphylococcus aureus, and to β-lactamases produced by some gram-negative organisms such as Enterobacter cloacae.

According to a further feature of the invention, we provide pharmaceutical compositions (including veterinary compositions) containing at least one of the acids, physiologically acceptable salts or metabolically labile esters according to the invention wherein B represents >S. The compositions may contain a further antibiotic. The compositions will normally also contain a pharmaceutical (including veterinary) carrier or excipient.

The compositions of the invention include those in a form adapted for oral or parenteral use.

The compositions may, for example, take the form of powders, tablets, capsules, lozenges, solutions and syrups suitable for oral administration, and may include, for example, starch, lactose, talc, magnesium stearate, gelatin, distilled water and suspending, dispersing, emulsifying, flavouring or colouring agents.

The compounds may be further formulated in rectal compositions such as suppositories or retention enemas.

The compounds of the invention may be formulated for parenteral administration e.g. for injection. The compounds may thus be presented in ampoules for formulation before use.

The active compounds of the invention will generally be administered to humans at a total daily dosage level of 50 mg to 20 g, preferably from 100 mg to 10 g, advantageously 250 mg to 5 g, which may be in divided doses given 1-4 times per day. Dosage units will in general contain 12.5 mg to 5 g, preferably 50 mg to 1 g of active compound according to the invention.

Active compounds of the invention may be of use in treating a variety of diseases in humans and animals, caused by pathogenic bacteria, such as respiratory tract or urinary tract infections.

The compounds of formula (I) may be prepared in a variety of ways as illustrated in the reaction diagram shown hereinafter.

The compounds of formula (I) wherein B represents $\gtrdot$S and $R^1$ represents an amino group may be prepared from a compound of formula (I) in which B represents $\gtrdot$S and $R^1$ represents an azido group by reduction, for example by catalytic hydrogenation. The hydrogenation catalyst is normally a noble metal catalyst, e.g. palladium, platinum or rhodium, or another transition metal catalyst such as nickel. The catalyst may be supported, e.g. on charcoal or kieselguhr; the metal catalyst is preferably palladium e.g. as 10% palladium on charcoal. Hydrogenation will desirably be effected in a solvent for the starting material.

Suitable solvents for the hydrogenation include ethers such as diethyl ether or tetrahydrofuran; esters such as ethyl acetate; ketones such as acetone or methyl ethyl ketone; chlorinated hydrocarbons such as methylene

chloride; amides such as dimethylformamide or dimethylacetamide; alcohols such as ethanol; or mixtures thereof. Such solvents may advantageously be admixed with water or an aqueous buffer. In some combinations a two phase system may result. In cases where the substrate is sufficiently water soluble, water or an aqueous buffer may be used alone.

The azido group may alternatively be reduced using dissolving metal reducing agents e.g. zinc and aqueous hydrochloric acid controlled in the pH range 2 to 6, preferably 4 - 4.5. Suitable solvents include, for example, water-miscible solvents such as ketones e.g. acetone, ethers such as tetrahydrofuran and alcohols such as ethanol.

Certain esters used as starting material may be cleaved during reduction, e.g. arylmethyl esters such as the benzyl and p-nitrobenzyl esters, to yield a free carboxyl group. Selective reduction of the azide group $R^1$ is possible where the ester group is not cleavable by reduction or only cleavable under different conditions from those used to reduce the azide group.

The amino acid product may be isolated from solution by, for example, partitioning between the solvent (where water-immiscible) and water and, after removal of catalyst, the aqueous phase may be lyophlised to yield the desired acid.

The acid product may also be isolated from solution by precipitation or crystallisation either as its zwitterionic form or as a salt as defined above. Depending on conditions the isolated compound may be in the form of a solvate or hydrate.

According to another aspect of the invention, the compounds of formula (I) in which $R^1$ represents $-N_3$ and B represents >S may be prepared from compounds of formula (II)

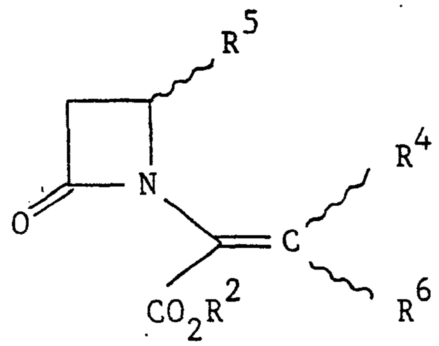

(II)

[wherein

$R^2$ represents a carboxyl esterifying group as defined above for $R^{2a}$;

$R^4$ represents a group of formula $-R^3N_3$ (where $R^3$ is as defined above);

$R^5$ represents a halogen atom or a group of formula $-\overset{\oplus}{N}R^xR^yR^zA^{\ominus}$ ( in which $R^x$, $R^y$ and $R^z$, which may be the same or different, each represents an aliphatic, araliphatic or aromatic group or two of $R^x$, $R^y$ and $R^z$ together with the nitrogen atom to which they are attached represent a five-, six- or seven-membered heterocyclic ring optionally containing a further heteroatom and the third of $R^x$, $R^y$ and $R^z$ is as defined above or $R^x$, $R^y$ and $R^z$ together with the nitrogen atom to which they are attached form an aromatic heterocyclic ring or an aromatic or non-aromatic polycyclic heterocyclic system and $A^{\ominus}$ represents an anion and

$R^6$ represents a leaving group, for example a halogen atom or an acylated or etherified hydroxy group] by reaction with hydrogen sulphide(where necessary in the presence of a base) or with a hydrosulphide or sulphide salt, followed, where a free acid or salt is

desired, by deesterification and salt formation, and where a sulphoxide is desired by oxidation.

Suitable hydrosulphide and sulphide salts include, for example, alkali metal, e.g. sodium, potassium and lithium salts; alkaline earth metal, e.g. calcium, salts; and onium salts, for example quaternary ammonium salts wherein the quaternary ammonium ion has the formula

$$R''-\overset{\displaystyle R'}{\underset{\displaystyle R'''}{N}}{}^{\oplus}-R$$

where R, R', R'' and R''' which may be the same or different each represents an alkyl or aralkyl group. Examples of such salts include the benzyltrimethylammonium and tetra-n-butylammonium salts.

In the reaction with hydrogen sulphide, the presence of a base, either organic or inorganic, is desirable to accelerate the reaction, to optimise yields and to curtail excessive acidity. It is in general preferred to use as weak a base as possible which is effective in the reaction. Suitable bases for use in the reaction include amine bases, for example, tertiary amine bases, e.g. trialkylamines, preferably those wherein the alkyl groups have from 1 to 4 carbon atoms such as triethylamine, hindered bases such as 3-azabicyclo-[3,2,2]-nonane or aromatic bases such as pyridine.

Where $R^5$ represents a halogen atom, the reaction will in general be effected at a temperature of from -150°C to +50°C, reaction temperatures of from -50°C to +10°C, particularly -15°C to +10°C, being preferred.

The reaction is desirably effected in the presence of an inert solvent, stable to the reaction conditions

employed. Suitable solvents include, for example, cyclic ethers e.g. tetrahydrofuran and dioxan, halogenated hydrocarbons, e.g. methylene chloride and 1,2-dichloroethane, esters, e.g. ethyl acetate and amide solvents, e g. dimethylformamide. Alternatively, where the reaction is effected in the presence of a base, an excess of base may serve as solvent.

Where $R^5$ represents a group of formula $-\overset{\oplus}{N}R^xR^yR^zA^{\ominus}$ reaction with hydrogen sulphide or a hydrosulphide or sulphide salt is preferably carried out at temperatures of from $-30^{\circ}$ to $+30^{\circ}C$. The reaction is completed by cyclisation to form an azido penem of formula (I) by heating. The cyclisation reaction is preferably effected above +40°C, e.g. at +50°C to +100°C.

Suitable solvents include those described above in relation to the process when $R^5$ is a halogen atom, provided that the solvent can be conveniently used at the temperature employed.

Following the reaction of the compound of formula (II) wherein $R^5$ represents a group of formula $-\overset{\oplus}{N}R^xR^yR^zA^{\ominus}$ with the hydrogen sulphide or hydrosulphide or sulphide salt, it may be possible to isolate a thioenolate of the formula (IIA)

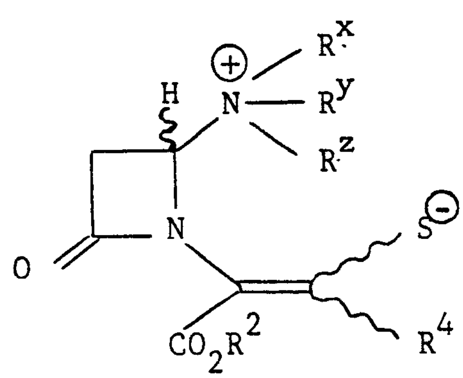

(IIA)

(wherein $R^x$, $R^y$, $R^z$, $R^4$ and $R^2$ have the above meanings). On heating, e.g. in a solvent such as ethyl acetate or 1,2-dichloroethane, the compound of formula (IIA) cyclises to the desired penem of formula (I).

Cyclisation may if desired be carried out in the presence of an acid scavenging agent, e.g. an insoluble inorganic base such as solid, anhydrous sodium, potassium or calcium carbonate.

In the compound of formula (II), when $R^5$ represents a halogen atom it is preferably a chlorine or bromine atom.

In the case that $R^5$ represents a group of formula $-\overset{\oplus}{N}R^xR^yR^zA^\ominus$ the anion $A^\ominus$ will generally be derived from the reagent used for the introduction of the group $R^6$ e.g. a halide ion such as a chloride ion, as described hereinafter.

Where $R^5$ represents a group of formula $-\overset{\oplus}{N}R^xR^yR^z A^\ominus$ $R^x$, $R^y$ and $R^z$ may, for example each represent an alkyl group having up to 8 carbon atoms, e.g. a $C_{1-6}$ alkyl group preferably a $C_{1-4}$ group, an aralkyl group having up to 6 carbon atoms in the alkyl portion or an aryl group, such aralkyl and aryl groups desirably being monocyclic, e.g. benzyl and phenyl, and such alkyl groups including also cycloaliphatic e.g. $C_{3-7}$ cycloalkyl groups; or two of $R^x$, $R^y$ and $R^z$ may together with the nitrogen atom to which they are attached represent a five-, six- or seven-membered heterocyclic ring optionally containing a further oxygen or sulphur atom e.g. a N-alkyl-piperidinium or N-alkyl-morpholinium group; or $R^x$, $R^y$ and $R^z$ may, together with the nitrogen atom to which they are

attached, represent a bicylic, non-aromatic heterocyclic ring system, e.g. a quinuclidinium group, or an aromatic ring e.g. pyridinium. It is preferred that $R^x$, $R^y$ and $R^z$ do not all represent bulky groups such as phenyl. A triethylammonium group is particularly preferred.

$R^6$ is preferably the residue of an acid $R^6H$ having a $pK_a$ (in water at 25°C) of 3.5 or less.

Where $R^6$ represents an acylated or etherified hydroxy group this may, for example, be an aliphatic, cycloaliphatic, aromatic or araliphatic carbonyloxy, sulphonyloxy or phosphinyloxy group containing 1 to 20 carbon atoms or an aliphatic ether group containing 1 to 6 carbon atoms, e.g. methoxy. Suitable sulphonyloxy groups include, for example, those wherein the aliphatic or aromatic grouping is an alkyl (e.g. $C_{1-8}$) group, which may be substituted by a halogen atom e.g. fluorine or chlorine, or an aryl (e.g. $C_{6-15}$) group which may carry alkyl, e.g. methyl, alkoxy e.g. methoxy or halogen e.g. bromine substituents. Suitable phosphinyloxy groups include, for example, groups of formula $-O\underset{R^8}{\overset{O}{P}}-R^7$ where $R^7$ and $R^8$, which may be the same or different, may each represent an alkyl, aryl or aralkyl group or may together with the phosphorus atom form a 5- or 6-membered ring. Alternatively $R^7$ and/or $R^8$ may represent groups $OR^a$ and $OR^b$ respectively where $R^a$ and $R^b$ are as defined for $R^7$ and $R^8$ or may together form a divalent 5- or 6-membered ring. An example of the latter type of phosphinyloxy group is the group

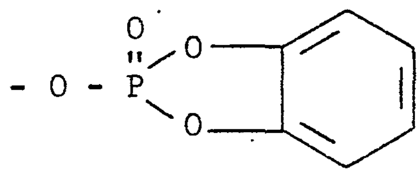

Suitable carbonyloxy groups include, for example, those wherein the aliphatic or aromatic grouping is an alkyl (e.g. $C_{1-8}$) group optionally substituted by one or more halogen atoms or an aryl (e.g. $C_{6-15}$) group optionally substituted by, for example, one or more halogen atoms or nitro groups.

When $R^6$ represents a halogen atom, this is preferably a chlorine or bromine atom.

Preferred meanings for $R^6$ include methanesulphonyloxy, trifluoromethanesulphonyloxy, p-toluenesulphonyloxy, benzenesulphonyloxy, dimethoxyphosphinyloxy groups and a chlorine atom.

The products of the ring-closure reaction will be esters of an azide compound of formula (I) wherein B represents a group >S. Where a free acid of formula (I) in which $R^1$ is an azido group or an amino group (or a salt of such an acid) is ultimately required, the ester should subsequently be cleaved at any stage after the ring-closure. Where an ester of a compound of formula (I) is required different from that originally produced, this may be obtained by deesterification followed by re-esterification as described hereinbelow.

Suitable methods for deesterification are well known in the art. Thus, for example, a silyl or stannyl ester may be cleaved by mild solvolysis e.g. by reaction

with water, alcohols, phenols or carboxylic acids e.g. acetic acid. Alternatively, esters which are readily subject to reduction, for example, arylmethyl esters such as benzyl, benzhydryl, trityl or o-nitrobenzyl or more preferably, p-nitrobenzyl esters can be cleaved by reduction e.g. by hydrogenolysis, for example using a metal catalyst, e.g. a noble metal such as platinum, palladium or rhodium. The catalyst may be supported e.g. on charcoal or kieselguhr.

In the case of nitrobenzyl e.g. p-nitrobenzyl esters, cleavage may also be effected by reduction of the nitro group (e.g. using a dissolving metal reducing agent such as zinc in acetic acid or zinc in aqueous tetrahydrofuran or acetone controlled in the pH range 3-6, preferably 4-4.5 e.g. by the addition of aqueous HCl; aluminium amalgam in a moist ether, e.g. tetrahydrofuran; or iron and ammonium chloride in an aqueous ether such as aqueous tetrahydrofuran) followed by hydrolysis either under the reduction conditions or by subsequent treatment with acid. Alternatively a p-nitrobenzyl ester may be cleaved under alkaline conditions using, for example, sodium sulphide in a solvent such as aqueous tetrahydrofuran, dimethylformamide or acetone. However, such reductive cleavage may, simultaneously, effect reduction of the $-N_3$ grouping to $-NH_2$, to produce a compound of formula (I) in which $R^1$ is $-NH_2$ and $R^{2a}$ is a hydrogen atom. The careful use of selective methods may, however, enable the azido acid of formula (I) to be prepared. Thus, for example, the p-nitrobenzyl ester of the azido acid may be deesterified by using a dissolving metal reducing

agent such as zinc and hydrochloric acid with pH control as described above to yield the azide acid or a salt thereof.

Where it is desired to produce a salt of the compound of formula (I), an acid may be reacted, e.g. in an appropriate organic solvent with an appropriate base, preferably in equimolar quantities and preferably under conditions favouring precipitation of the salt. In the formation of alkali metal salts for example, e.g. sodium or potassium salts, an alkanoate is a preferred base e.g. a 2-ethyl hexanoate. Acid addition salts of the invention may be produced by reaction of an amino compound of formula (I) with an appropriate acid.

Esters of the acid of formula (I) in which $R^1$ is an azido group may, for example, be prepared by reaction with an alcohol, phenol, silanol or stannanol or a reactive derivative thereof to form the desired ester. Reaction will desirably be effected under mild conditions in order to prevent rupture of the bicyclic nucleus. The use of neutral or mild acidic or basic conditions therefore, at temperatures between -70° and +35°C is preferred.

The alkyl, alkoxyalkyl and aralkyl esters may be prepared by reaction of the acid of formula (I) with the appropriate diazoalkane or diazoaralkane e.g. diazomethane or diphenyldiazomethane. The reaction will generally be effected in an ether, ester or a halohydrocarbon solvent, e.g. diethyl ether, ethyl acetate or dichloromethane. In general, reduced temperatures are preferred, for example, -15° to +15°C.

The esters derived from alcohols may also be produced by reaction of a reactive derivative of the alcohol, for example, a halide such as the chloride, bromide or iodide or a hydrocarbonsulphonyl derivative such as a methanesulphonyl or *p*-toluenesulphonyl ester, with a salt of the acid of formula (I) e.g. an alkali or alkaline earth metal salt such as a lithium, sodium, potassium, calcium or barium salt or an amine salt, such as a triethylammonium salt. This reaction is preferably carried out in a substituted sulphoxide or amide solvent e.g. dimethyl sulphoxide, dimethylformamide or hexamethylphosphoramide. Alternatively, the esters may be prepared by reaction of the acid with the alcohol in the presence of a condensing agent such as dicyclohexylcarbodiimide.

Stannyl esters may conveniently be formed by reaction of the carboxylic acid of formula (I) or a salt thereof with reactive tetravalent tin moieties. Trialkyl tin oxides are preferred for the synthesis of tin compounds in view of their availability and low toxicity.

Silyl esters of the acid of formula (I) may be formed by reaction with a reactive tetravalent silicon moiety. Trialkylsilyl halides and mono- or bis-silylated acetamides are preferred as silylating agents. Proton acceptors e.g. weak bases such as pyridine may often be used with advantage when hydrogen halides are formed during such esterification.

It should be noted that an azido acid of formula (I) ($R^1$=$N_3$; $R^{2a}$=H) may, if desired, be re-esterified to introduce an ester group different from that initially present in the azide ester product prepared from the

compound of formula (II). It is thus possible to introduce an ester group which is inert to the conditions used in subsequent reduction of the azido group to $-NH_2$, thereby facilitating production of esters of the amine acid of formula (I) ($R^1$=$NH_2$; $R^{2a}$=H). Such esters may be of a type which are not removable by reductive cleavage, e.g. alkyl esters such as the methyl ester or the acetoxymethyl ester or they may be susceptible to reductive cleavage but be less reactive than the azido group.

Esters of the acid of formula I in which $R^1$ is an amino group may be prepared by conventional means used in relation to the preparation of esters of amino acids.

Compounds of general formula (I) in which $R^1$ represents $-N_3$ and B represents $>S\rightarrow O$ may be prepared from corresponding compounds of general formula (I) in which $R^1$ represents $-N_3$ and B represents $>S$ by oxidation, for example, with peracids such as peracetic, monoperphthalic, m-chloroperbenzoic or metaperiodic acid. Oxidation is preferably effected at a temperature not greater than $10^oC$ and avoiding excess oxidising agent.

Compounds of general formula (I) in which $R^1$ represents $-NH_2$ and B represents $>S\rightarrow O$ may be prepared from compounds of general formula (I) in which $R^1$ represents $-N_3$ and B represents $>S\rightarrow O$ by selective reduction using mild reducing agents capable of reducing $-N_3$ to $-NH_2$ without reducing the sulphoxide grouping.

The compounds of general formula (II) (used as starting materials) may be prepared by a variety of methods. Thus they may, for example, be prepared by reaction sequences involving clavulanic acid and its

derivatives or they may be prepared by total synthesis.

One method of preparing compounds of general formula (II) wherein $R^5$ represents a halogen atom involves reacting a compound of formula (III)

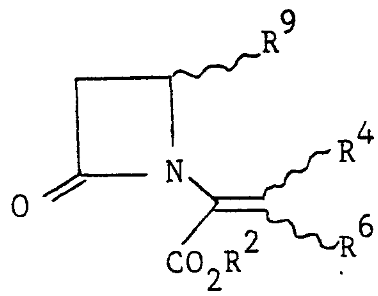

(III)

(wherein $R^2$, $R^4$ and $R^6$ are as hereinbefore defined and

$R^9$ is a group of formula $-SR^{10}$ or $-\overset{O}{\overset{\uparrow}{S}}-R^{10}$

where $R^{10}$ is an aliphatic, araliphatic, aromatic or heterocyclic group) with an electrophilic halogenating agent.

The halogenating agent may, for example, be molecular halogen,an N-halo amide or imide, or an interhalogen compound. The N-halo amide or N-halo imide may include a cyclic system, the amide or imide linkage forming part of the cyclic system; examples of such N-halo amides include N-bromo-caprolactam and examples of such N-halo imides include the 1,3-dibromo-5,5-di-(lower alkyl) hydantions (e.g. 1,3-dibromo-5,5-dimethyl-hydantion); N-bromosuccinimide; N-chlorosuccinimide; N-bromophthalimide etc. Other useful N-halo amides include N-halo lower alkanoamides e.g. N-bromoacetamide. The interhalogen compound may be iodinemonochloride.

The reaction will preferably be carried out in an inert solvent, e.g. a halogenated hydrocarbon, for example methylene chloride.

Molecular halogen, e.g. chlorine or bromine, may, for convenience, be added in solution, e.g. in a halogenated hydrocarbon, such as carbon tetrachloride.

Suitable reaction temperatures are from -150°C to 25°C. The use of low temperature e.g. below about 0°C, has been found advantageous in assisting the control of the reaction.

Where molecular halogen is used, it may be desirable to carry out the reaction in the presence of an acid binding agent in order to remove any hydrogen halide as it is formed and thus prevent side reactions. Suitable acid binding agents include amides e.g. carboxylic acid amides such as acetamide, metal carbonates e.g. calcium carbonate, oxiranes e.g. propylene oxide, or molecular sieves.

The acid binding agent, when used, will desirably be present in a quantity which ensures as rapid removal of hydrogen halide as possible.

When $R^{10}$ represents an aliphatic group, this may be saturated or unsaturated and thus may, for example, be an alkyl or alkenyl group which may contain 1-6 carbon atoms e.g. $C_{1-4}$ alkyl such as methyl or butyl, or a cycloalkyl group, which may have 3-7, preferably 5 or 6 carbon atoms. Such alkyl groups may, optionally, carry one or more etherified hydroxy groups.

When $R^{10}$ is an araliphatic or aromatic group, it will desirably be an aralkyl group which may have 1-6

carbon atoms in the alkyl portion, or an aryl group, the rings in such aryl and aralkyl groups, preferably being monocyclic, e.g. phenyl; and optionally carrying substituents such as $C_{1-4}$ alkyl groups e.g. methyl groups.

When $R^{10}$ is a heterocyclic group, it will desirably contain a carbon-attached 5-7 membered heterocyclic ring containing one or more heteroatoms such as nitrogen, sulphur or oxygen, which may be aromatic e.g. pyridyl and/or carry one or more alkyl groups, for example containing 1-6 carbon atoms e.g. a methyl group or may have a bicyclic structure, an example of such a group being a benzothiazol-2-yl group.

Specific groups $R^9$ which may be mentioned include, for example, methylsulphinyl; p-tolylthio; $C_{1-4}$ alkylthio e.g. n-butylthio, methylthio and ethylthio and benzylthio groups.

Compounds of formula (III) may, for example, be prepared from compounds of formula (IV),

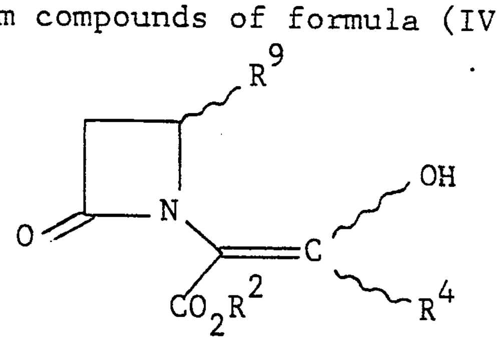

(IV)

(wherein $R^2$, $R^4$ and $R^9$ are as hereinbefore defined) by reaction with one or more reagents serving to replace the enolic hydroxy group by, or convert the enolic hydroxy group into, a leaving group e.g. a halogen atom or an acyloxy or ether group.

Compounds of formula (III) in which $R^6$ represents a halogen atom may, for example, be prepared by treating a compound of formula (IV) with dimethylformamide and phosphorus trichloride at a temperature of from -20° to +40°C.

Compounds of formula (III) in which $R^6$ is an acyloxy group may be prepared by reacting a compound of formula (IV) with an appropriate acylating agent, e.g. an acyl halide or anhydride. Such an acyl halide will desirably be a phosphinyl or sulphonyl halide, the phosphinyl and sulphonyl portions being as defined above. Thus, in this case, the compound of formula (IV) may be reacted with an alkane sulphonyl halide or aryl sulphonyl halide, for example methanesulphonyl halide or p-toluene sulphonyl halide, or a dialkoxyphosphinyl chloride e.g. dimethoxyphosphinyl chloride. Reaction is normally carried out in a solvent, e.g. an ether such as diethyl ether or tetrahydrofuran or a halogenated hydrocarbon such as methylene chloride. Reaction temperatures are generally from -30° to +30°C, preferably from -15° to +20°C.

The reaction will normally be effected in the presence of an acid binding agent, e.g. a tertiary amine such as triethylamine, pyridine or collidine or an oxirane such as propylene oxide.

Compounds of formula (III) in which $R^6$ is an ether group may, for example, be prepared from compounds of formula (IV) by reaction with a diazoalkane, e.g. diazomethane, optionally in the presence of a Lewis acid catalyst such as boron trifluoride etherate. The reaction may be effected in a solvent such as an ether e.g. diethyl

ether, dioxan or tetrahydrofuran, a hydrocarbon e.g. light petroleum fraction, or a halogenated hydrocarbon e.g. dichloromethane or chloroform. The reaction temperature is preferably low, e.g. -15° to +15°C.

Alternatively a compound of formula (II) where $R^6$ is a leaving group and $R^5$ represents a halogen atom may be prepared from a compound corresponding to formula (II) but where $R^6$ is hydroxy, by reaction with one or more reagents serving to replace the hydroxy group by or convert it into a leaving group, e.g. a halogen atom or an acyloxy or ether group. Such reagents may be of the type previously described in relation to compounds of formula (IV). For acylation an acid binding agent will normally be present, e.g. an oxirane such as propylene oxide or a base; where a base is used, this is preferably a weak base such as pyridine.

The compounds corresponding to formula (II) in which $R^5$ is halogen but in which $R^6$ is hydroxy may be prepared from compounds of formula (IV) by reaction with an electrophilic halogenating agent e.g. of the type described above in relation to the compounds of formula (III).

The compounds of formula (IV) wherein $R^9$ represents a group of formula $-SR^{10}$ as defined above and $R^4$ represents a group of formula $-CH_2CH_2N_3$ may be prepared from compounds of formula (V)

readily prepared from compounds of formula (V)

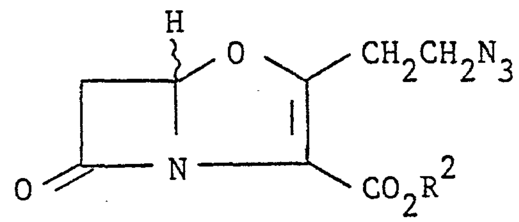

(V)

(wherein $R^2$ is as defined above) by reaction thereof with a thiol $R^{10}SH$, wherein $R^{10}$ is as defined above.

The thiol $R^{10}SH$ will preferably be present in excess as compared with the starting material of formula (V).

The reaction will desirably be carried out in an aprotic organic solvent, e.g. an ether solvent such as tetrahydrofuran although the thiol may itself serve as the medium for the reaction. The reaction will desirably be carried out at or above ambient temperature, e.g. at from +20° to +80°C.

Compounds of formula (II) wherein $R^5$ represents a group of formula $-\overset{\oplus}{N}R^xR^yR^zA^\ominus$ may be prepared by reaction of compounds of formula (VII)

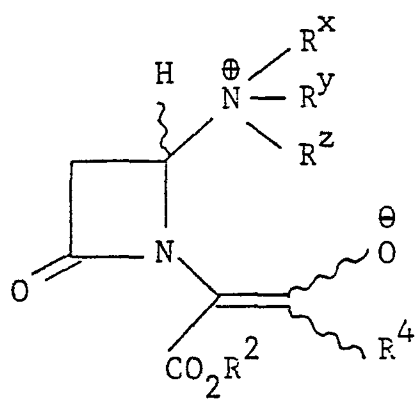

(VII)

(where $R^2$, $R^4$, $R^x$, $R^y$ and $R^z$ have the above meanings) by reaction with an appropriate reagent serving to introduce the group $R^6$. The introduction of the group $R^6$ may be effected by the procedures described above for the formation of compounds of formula (III). In

particular, the compound of formula (VII) may be reacted with a sulphonylating agent e.g. a methanesulphonylating agent such as methanesulphonyl chloride to form a product of formula (II) in which $R^6$ is methanesulphonyloxy. In general, as mentioned above, an anion $A^{\ominus}$, derived from the reagent used for introduction of the group $R^6$, e.g. a halide ion such as a chloride ion will be associated with the compound of formula (II) thus formed. The reaction may be effected in the presence of a base for example a tertiary base such as pyridine. The latter may serve as solvent or a further solvent may be present, e.g. a halogenated hydrocarbon such as methylene chloride or 1,2-dichloroethane.

If desired such a compound of formula (VII) may be used in the presence of pyridine to prepare a compound of formula (II) in situ which compound may then be directly cyclised to a compound of formula (I) by addition of triethylamine and passing in hydrogen sulphide. Heating, e.g. to about 70°C completes the reaction. Where a compound of formula (IIA) is prepared from a compound of formula (VII) without isolation of the compound of formula (II), the compound of formula (IIA) may be partly in the form of an acid addition salt with an acid HA (where A is as defined above) such as HCl.

Compounds of formula (VII) may, for example, be prepared by reaction of a compound corresponding to formula (II) (in which $R^5$ is halogen but in which $R^6$ is a hydroxy group) with a tertiary base $NR^xR^yR^z$ in the presence of a solvent such as ethyl acetate at temperatures of from 0 to 30°C, preferably at ambient temperature.

Compounds of formula (VII) in which $R^4$ represents a group of formula $-CH_2CH_2N_3$ may alternatively be prepared by reaction of a compound of formula (VI)

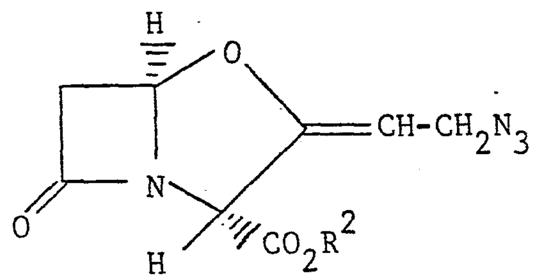

(VI)

(wherein $R^2$ has the above meaning) with a base $NR^xR^yR^z$ at relatively low temperature. Where $NR^xR^yR^z$ is to be the residue of a weak base such as pyridine, the ring-opening may be effected by reaction with the weak base, in the presence of a catalytic quantity of a strong base.

Compounds of formula (V) will desirably be prepared from compounds of formula (VII) by heating e.g. at a temperature of from +50°C to +100°C in a suitable solvent. Suitable solvents include esters e.g. ethyl acetate, ketones, e.g. acetone, ethers e.g. tetrahydrofuran and halogenated hydrocarbons e.g. 1,2-dichloroethane and chloroform.

Alternatively, the compounds of formula (V) may be prepared from compounds of formula (VI) by treatment thereof at elevated temperature with a base.

Preferred bases for use in this reaction are tertiary organic bases e.g. tertiary amines. Such amines will desirably have the formula $R^xR^yR^zN$, where $R^x$, $R^y$ and $R^z$, are as defined above.

Use of simpler trialkylamines e.g. having 1-6 carbon atoms in each alkyl group, especially methyl, ethyl, propyl or butyl groups, is preferred and triethylamine is especially preferred.

Reaction will desirably be carried out in a suitable inert solvent. Such solvents will preferably include esters e.g. ethyl acetate, ethers e.g. tetrahydrofuran, ketones e.g. acetone, amides e.g. dimethylformamide and halogenated hydrocarbons e.g. 1,2-dichloroethane or chloroform.

Reaction will desirably be effected under reflux, a temperature of from 50° to 100°C being preferred.

Where necessary, compounds of formula (V) may be purified by conversion to compounds of formula (VII) by treatment with a base $NR^xR^yR^z$ at relatively low temperature and reconversion to compounds of formula (V) by heating as described hereinabove.

Compounds of formula (VI), useful as starting materials, may be prepared by reaction of esters of (3R,5R,Z)-2-(2-chloro- or bromo-ethylidene)-clavam-3-carboxylic acid with sodium azide.

Alternatively compounds of general formula (IV) may be obtained by reaction of a compound of formula (VIII),

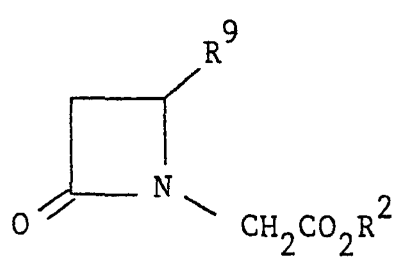

(VIII)

(wherein $R^2$ and $R^9$ are as defined above with a compound of formula (IX),

$$R^4CO\ Hal \qquad (IX)$$

(wherein $R^4$ is as defined above and Hal represents a halogen, e.g. chlorine, atom) in the presence of a strong base, e.g. lithium di(trimethylsilyl)amide, preferably in the presence of a solvent such as tetrahydrofuran.

Compounds of formula (VIII) may be formed, for example, from a compound of formula (X)

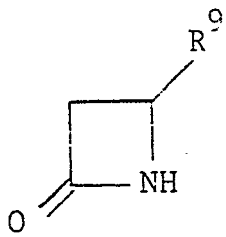

(X)

(wherein $R^9$ is as defined above) by reaction with a compound of formula (XI)

$$Hal\ CH_2CO_2R^2 \qquad (XI)$$

(wherein $R^2$ is as defined above and Hal is as defined above e.g. a bromine atom) in the presence of a base, e.g. potassium carbonate or sodium hydride, preferably in the presence of an anhydrous organic solvent such as dimethylformamide. Compounds of formula (X) may be obtained, for example, from a compound of formula (XII),

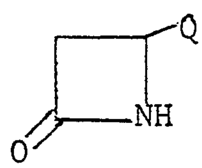

(XII)

(where Q represents a leaving group preferably an acylated hydroxy group such as exemplified hereinbefore in connection with $R^6$) by reaction with a salt of an appropriate compound of formula $HR^9$.

Compounds of general formulæ (VIII) and (X) wherein $R^9$ is $-SR^{10}$ may, if desired, be converted into their corresponding sulphoxides by oxidation, for example, using a peracid such as e.g. peracetic acid, monoperphthalic acid, m-chloroperbenzoic acid or metaperiodic acid, preferably in the presence of an organic solvent such as acetic acid or dichloromethane. Preferred temperatures are not greater than $10^oC$ e.g. about $0^oC$. Alternatively oxidation to form the sulphoxide may be effected at a later stage in the reaction sequence.

Compounds of general formulae (IX) and (XI) are known from the literature.

The compounds of general formulae (VIII) and (X) are described in Belgian Patent Specification Nos. 772,940; 755,940 and 772,990 and South African Patent Specification No.77/0435. The compounds of formula (II), (IIA), (III), (IV) (V) and (VII) and compounds corresponding to formula (II) in which $R^5$ is halogen but wherein $R^6$ is hydroxy are in general believed to be novel compounds and thus according to a further feature of the invention there are provided compounds of the general formula (XIII),

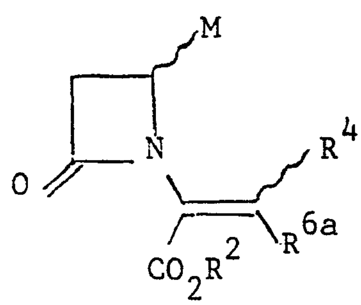

(XIII)

wherein $R^4$ is as hereinbefore defined; $R^2$ represents a carboxyl esterifying group; and either M represents a halogen atom or a group $R^9$ (in which $R^9$ represents a group $-SR^{10}$ or $-\overset{O}{\uparrow}{S}-R^{10}$ where $R^{10}$ is an aliphatic, araliphatic, aromatic

or heterocyclic group) and $R^{6a}$ represents a hydroxy group or a leaving group;

or M represents a group of formula

$$-\overset{\oplus}{N}R^xR^yR^zA^{\ominus}$$

(where $R^x$, $R^y$, $R^z$ and $A^{\ominus}$ are as hereinbefore defined) and $R^{6a}$ represents a leaving group;

or M represents a group $\overset{\oplus}{N}R^xR^yR^z$ (where $R^x$, $R^y$ and $R^z$ are are as hereinbefore defined) and $R^{6a}$ represents $S^{\ominus}$ or $O^{\ominus}$.

Processes for the preparation of the compounds embraced by formula (XIII) (such processes being as set out hereinbefore) constitute yet further features of the present invention.

The foregoing reactions are illustrated in the following diagram.

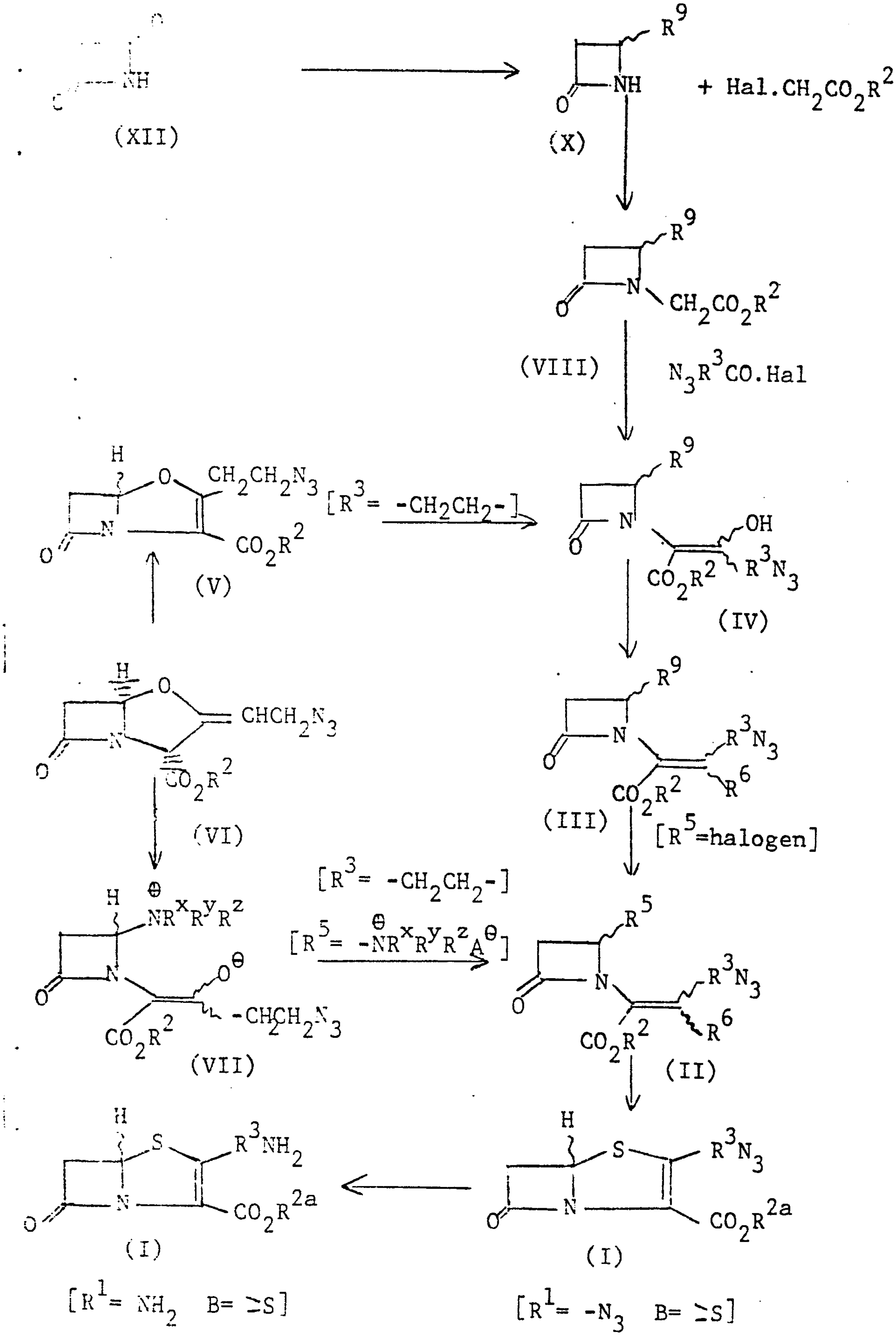
(XII)
(X)
+ Hal.$CH_2CO_2R^2$
(VIII)
$N_3R^3$CO.Hal
(V)
[$R^3$= $-CH_2CH_2-$]
(IV)
(VI)
(III)
[$R^5$=halogen]
(VII)
[$R^3$= $-CH_2CH_2-$]
[$R^5$= $-\overset{\ominus}{N}R^xR^yR^zA^{\ominus}$]
(II)
(I)
(I)
[$R^1$= $NH_2$ B= $\geq$S]
[$R^1$= $-N_3$ B= $\geq$S]

The following non-limiting Examples serve to illustrate compounds of the present invention and processes for their preparation.

In the Examples, certain bicyclic compounds are named with reference to "penam", the conventional name given to the heterocycle of formula (XIII),

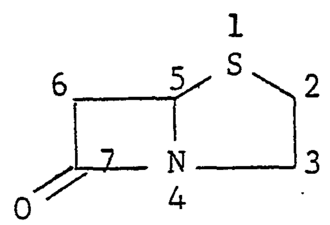

(XIII)

or "clavam", the name given to the heterocycle of formula (XIV)

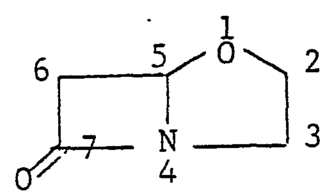

(XIV)

Example 1

4-Azido-1-(4-nitrobenzyloxycarbonyl)-1-(2-oxo-4-triethylammonioazetidin-1-yl)but-1-en-2-olate

A solution of 4-nitrobenzyl (3R,5R,Z)-2-(2-azidoethylidene)-clavam-3-carboxylate (0.377 g) and triethylamine (0.30 ml) in ethyl acetate (3.5 ml) was stood at 28° for 40 minutes. The resulting solid was collected and dried *in vacuo* to give the title salt (0.092 g), $\nu_{max}$ (Nujol) 2100 ($N_3$), 1765 and 1750 (β-lactam), 1652 ($CO_2R$), 1520 and 1348 $cm^{-1}$ ($NO_2$) τ (DMSO-$d_6$) values for *ca.* 1:1 mixture of isomers include 1.77 (d, J 8Hz, aromatic protons 2.38 + 2.40 (d, J 8Hz, aromatic protons), 4.46 + 4.63 (m, azetidinyl C-4 H), 4.84 + 4.73 and 4.96 (s + ABq, J 14Hz, benzylic protons), 8.80 + 8.88 (t, J 7Hz, $N(CH_2CH_3)_3$).

Example 2

4-Nitrobenzyl 2-(2-azidoethyl)clav-2-em-3-carboxylate

A suspension of 4-azido-1-(4-nitrobenzyloxycarbonyl)-1-(2-oxo-4-triethylammonioazetidin-1-yl)but-1-en-2-olate (0.116 g) in dry chloroform (30 ml) was heated gently under reflux for 10 minutes. The resulting solution was cooled to 5° and then poured into petroleum ether (40-60°) (50 ml) with stirring. The resulting suspension was filtered and the filtrate evaporated to give the *title ester* (0.039 g), $\nu_{max}$ ($CHBr_3$) 2140 ($N_3$), 1802 (β-lactam), 1730 ($CO_2R$), 1524 and 1346 $cm^{-1}$ ($NO_2$), τ ($CDCl_3$) 1.78 and 2.40 (doublets, J 8Hz, aromatic protons), 4.02 (d, J 3Hz, C-5 H), 4.56 + 4.78 (ABq, J 14Hz benzylic protons), 6.15 (dd, J 17 and 3Hz, C-6 proton), 6.50 [obscured] (m, 6.5 to 6.9 (m, $-\underline{CH_2}\underline{CH_2}N_3$).

Example 3

4-Nitrobenzyl(5RS)-2-(2-azidoethyl)pen-2-em-3-carboxylate

Methanesulphonyl chloride (2.12 g) was added to a stirred suspension of 4-azido-1-(4-nitrobenzyloxycarbonyl)-1-(2-oxo-4-triethylammonioazetidin-1-yl)but-1-en-2-olate (2.846 g) in dry 1,2-dichloroethane (90 ml) containing pyridine at 5°. The reaction mixture was allowed to warm to room temperature and after 35 minutes was cooled to 5°. Hydrogen sulphide was passed through the stirred solution under nitrogen, and an excess of triethylamine was added in portions over 10 minutes. The resulting mixture was diluted with 1,2-dichloroethane (100 ml) and heated to boiling for 5 minutes. The solution was cooled to ambient temperature and washed with saturated brine containing 0.5N aqueous hydrochloric acid (twice), followed by saturated brine, and then dried over magnesium sulphate. The filtered solution was evaporated to low volume, silica gel was added, and the resulting slurry was evaporated to dryness. The impregnated silica gel was placed on top of a dry silica gel column and eluted with ether-petroleum ether (40-60°) mixtures. Appropriate fractions were combined and evaporated to afford the title ester (1.36 g) $\lambda_{max}$ ($CHCl_3$) 318nm (ε 6500), $\nu_{max}$ ($CHBr_3$) 2100 ($N_3$), 1786 (β-lactam), 1705 (ester), 1518 and 1344 $cm^{-1}$ ($NO_2$), τ ($CDCl_3$) 1.78 and 2.38 (doublets, J 9 Hz, aromatic protons), 4.31 (dd, J 4 and 2Hz, C-5 H), 4.54 and 4.78 (ABq, J 14Hz, benzylic protons), 6.15 and 6.49 (dd, J 16 and 4Hz, and dd, J 16 and 2Hz, C-6 protons), 6.50 (m, $-C\underline{H}_2N_3$), 6.6 to 7.2 (m, $-C\underline{H}_2CH_2$).

Example 4

(5RS)-2-(2-Aminoethyl)pen-2-em-3-carboxylic acid

A solution of 4-nitrobenzyl (5RS)-2-(2-azidoethyl)-pen-2-em-3-carboxylate (1.15 g) in ethyl acetate: ethanol (1:1; 50 ml) containing 10% palladium on charcoal (6.9 g) was shaken and hydrogenated at room temperature and at 1 atmosphere pressure. After 40 minutes the mixture was diluted with water (40 ml) and shaken for 5 minutes. The mixture was filtered through kieselguhr and the pad was washed with a little water followed by ethyl acetate. The aqueous phase was rewashed with ethyl acetate and then freeze-dried. The resulting foam was dissolved in water (2 ml) and, with stirring, was diluted with acetone (50 ml). The resulting solid was washed with acetone and then collected and dried <u>in vacuo</u> to afford the <u>title acid</u> (0.103 g), $\lambda_{max}$ (pH 6 buffer) 303nm ($\varepsilon$ 4000), $\nu_{max}$ (Nujol) 1753 ($\beta$-lactam), 1580 $cm^{-1}$ ($CO_2^-$), $\tau$ ($D_2O$ + DMSO-$d_6$) 4.30 (dd, J 3 and 1Hz, C-5 H), 6.20 and 6.53 (dd, J 16 and 3Hz, and dd, J 16 and 1Hz, C-6 protons), 6.82 (m, $-C\underline{H}_2NH_2$), 6.8 to 7.2 (m, $-C\underline{H}_2CH_2$).

Example 5

(4RS)-4-p-Tolylthioazetidin-2-one

Sodium hydride (80% suspension in oil, 5.157 g, 0.17 mole) was carefully added portionwise to a solution of p-thiocresol (17.76 g. 0.155 mole) in methanol (80 ml). The solution was then cooled to 5° and a solution of 2-acetoxyazetidin-4-one (18.133 g. 0.14 mole) in methanol (50 ml) was added. After 10 minutes a suspension formed which was poured into a stirred mixture of 2N hydrochloric acid (200 ml) and ethyl acetate (1 l.). The organic phase was separated, washed with brine, and dried, and evaporated. The residue was triturated with dichloromethane: petrol (1:10; 150 ml) and the solid collected, washed once with dichloromethane: petrol (1:20; 150 ml) and dried in vacuo to give the title compound (20.7 g). A similar sample had m.p. 102 to 103° and $\lambda_{max}$ (EtOH) 222.5 nm ($\varepsilon$ 10.650) and 254 nm ($\varepsilon$ 4.950).

Example 6

p-Nitrobenzyl (4'RS)-2-(2'-oxo-4'-p-tolylthioazetidin-1'-yl)acetate

Sodium hydride (80%, 0.330 g, 11 mmole) was added to a cooled (-20°) and stirred solution of the product of Example 5 (1.93 g, 10 mmole) and p-nitrobenzyl bromoacetate (2.74 g, 10 mmole) in dry DMF (40 ml). The solution was allowed to warm to ca 23° over 1 hour and then poured into a stirred mixture of ethyl acetate (200 ml) and 2N hydrochloric acid (200 ml). The aqueous phase was separated and extracted with ethyl acetate (200 ml). The combined organic solutions were washed with water (2 x 100 ml), and brine (100 ml), dried and evaporated to a gum.

This gum was purified by chromatography (B) (0 to 2% ether in dichloromethane) and appropriate fractions were combined and evaporated in vacuo to give the title compound (2.63 g).

A sample purified by preparative layer chromatography (dichloromethane:ether (20:1)) gave $\lambda_{max}$ (EtOH) 258 nm ($E^{1\%}_{1cm}$ 350).

Example 7

p-Nitrobenzyl (4'RS)-4-azido-3-hydroxy-2-(2'-oxo-4'-p-tolylthioazetidin-1'-yl)but-2-enoate

A stirred and cooled (0°) suspension of potassium azidoacetate (0.164 g) in dry ether (3 ml) was treated successively with oxalyl chloride (0.1 ml) and a drop of dry N,N-dimethylformamide. The suspension was stirred at 0° for 40 minutes.

n-Butyl lithium in hexane (1.52 molar solution, 1.15 ml) was added dropwise under nitrogen to a cooled (-78°)

solution of hexamethyldisilazane (0.37 ml) in dry tetrahydrofuran (10 ml). A solution of p-nitrobenzyl (4'RS)-2-(2'-oxo-4'-p-tolylthioazetidin-1'-yl) acetate (0.386 g) in dry tetrahydrofuran (10 ml) was then added followed, after 2 minutes, by the solution of azidoacetyl chloride described above. After stirring for 20 minutes at -78° the mixture was poured into a mixture of ethyl acetate and 0.5 N hydrochloric acid (100 ml of each).

The organic layer was washed with water, and brine (40 ml of each) and dried and the solvent was removed. The residue was purified by preparative layer chromatography using dichloromethane:acetone (20:1) for development.

The appropriate band was removed and extracted into acetone (100 ml) and the extract was evaporated to dryness. The residue was suspended in ether (20 ml), the suspension was stirred for 5 minutes and the insoluble material was filtered off, and washed with ether (2 x 5 ml), and dried to give the title ester (0.107 g) as a solid m.p 236° (decomp), $\lambda_{max}$ (EtOH) 277 nm ($E^{1\%}_{1cm}$ 470) with an inflection at 262 nm ($E^{1\%}_{1cm}$ 417) and $\nu_{max}$ (Nujol) 2135 and 2105 (azide), 1748 $cm^{-1}$ (β-lactam).

## Example 8

**p-Nitrobenzyl (2'RS)-4-azido-3-methanesulphonyloxy-2-(2'-chloro-4'-oxoazetidin-1'-yl)but-2-enoate**

A stirred and cooled (0°) solution of the product of Example 7 (1.36 g ) in dry dichloromethane was treated successively with methanesulphonyl chloride (0.34 ml) and triethylamine (0.60 ml).

After 20 minutes 0.5N hydrochloric acid (30ml) was added and the mixture was stirred for a further 5 minutes. The organic phase was separated, washed with water, and

brine (20 ml of each) and dried.

A solution of chlorine in dichloromethane (8.2 ml of a 5% solution, equivalent to 5.8 mmole of chlorine) was added to the above cooled (0°) solution.

The mixture was stirred at 0° for 20 minutes, the solvent was evaporated off and the residue was purified by column chromatography on kieselgel (30g). The column was eluted with toluene;ethyl acetate (20:1) and the appropriate fractions were combined and the solvent removed to give the title compound (0.548 g) as a gum, $\lambda_{max}$ (CHCl$_3$) 262.5nm ($E^{1\%}_{1cm}$ 342) and $\nu_{max}$ (CHBr$_3$) 2100 (azide), 1790 cm$^{-1}$ (β-lactam).

## Example 9

p-Nitrobenzyl (5RS)-2-azidomethyl-pen-2-em-3-carboxylate

Hydrogen sulphide (1 ml of a 1.4 molar solution in N,N-dimethylformamide) was added to a stirred and cooled (-20°) solution of triethylamine (0.26 ml) in dry N,N-dimethylformamide (20 ml), under nitrogen.

A few minutes later a cooled (-20°) solution of the product of Example 8 (0.43 g) in dry N,N-dimethylformamide (20 ml) was added and the mixture was stirred for a further 20 minutes at -20°. The reaction mixture was poured into a stirred mixture of ethyl acetate and N hydrochloric acid (40 ml of each), and the organic layer was separated and washed with water, and brine (20 ml of each). The organic solution was dried and the solvent removed to give an orange oil which was triturated with ethyl acetate: ether (1:5) (10 ml). The precipitate was filtered off and discarded. The filtrate was evaporated to an orange oil which was partitioned between ethyl acetate and N hydrochloric acid (100 ml of each). The organic phase was separated, and washed with water, and brine (30 ml of

BAD ORIGINAL

each) and dried and the solvent removed to give an orange foam (0.2 g).

This foam was subjected to preparative layer chromatography using toluene:ethyl acetate (4:1), for development. The appropriate band was extracted into ethyl acetate (50 ml) and the extract was evaporated to dryness to give the title compound (0.03 g) as a gum; $\lambda_{max}$ ($CHCl_3$) 269.5 nm ($E^{1\%}_{1cm}$ 417) with an inflection at 320 nm ($E^{1\%}_{1cm}$ 128) and $\nu_{max}$ ($CHBr_3$) 2110 (azide), 1788 $cm^{-1}$ (β-lactam).

Example 10

4-Azido-1-(4-nitrobenzyloxycarbonyl)-1-(2-oxo-4-triethylammonioazetidin-1-yl)but-1-en-2-olate

Triethylamine (11.1 ml) was added to a stirred solution of 4-nitrobenzyl (3R,5R,Z)-2-(2-azido-ethylidene)clavam-3-carboxylate (7.18 g) in ethyl acetate-ether (1:5, 120 ml). After 1½ hours the resulting solid was collected, washed with ether, and dried *in vacuo* to give the *title salt* (6.835 g), $\nu_{max}$ (Nujol) 2105 ($N_3$), 1778 (β-lactam), 1660 ($CO_2R$), 1525 and 1350 $cm^{-1}$ ($NO_2$), τ (DMSO-$d_6$) values for *ca.* 1:1 mixture of isomers include 1.77 (d, J 8 Hz, aromatic protons), 2.38 + 2.40 (doublets, J 8 Hz, aromatic protons), 4.46 + 4.63 (multiplets, azetidinyl C-4 H), 4.84 + 4.73 and 4.96 (s + ABq, J 14 Hz, benzylic protons), 8.80 + 8.88 (triplets, J 7 Hz, $N(CH_2CH_3)_3$).

Example 11

4-Nitrobenzyl (5RS)-2-(2-azidoethyl)pen-2-em-3-carboxylate

Methanesulphonyl chloride (12.15 g) was added to a stirred suspension of the product of Example 10 (16.26 g) in 1,2-dichloroethane (515 ml) containing pyridine (14.2 ml) and maintained at 0 to 5°. The reaction mixture was allowed to warm to room temperature and after 25 minutes was cooled to 0°. Hydrogen sulphide was passed through the stirred solution under nitrogen, and an excess of triethylamine was added in portions over 10 minutes. The resulting

mixture was diluted with 1,2-dichloroethane (350 ml) and heated to boiling for 5 minutes. The solution was cooled to ambient temperature and washed successively with 0.5 N aqueous hydrochloric acid (twice), 0.5 N aqueous sodium hydrogen carbonate, water, and saturated brine and then dried over magnesium sulphate. The filtered solution was evaporated to low volume, silica gel was added, and the resulting slurry was evaporated to dryness. The impregnated silica gel was transferred to the top of a dry silica gel column and eluted with ethyl acetate-petroleium spirit (40 - 60°) (1:3). Appropiate fractions were combined and evaporated to give an oil (4.21 g) which crystallised on addition of ethyl acetate. The resulting solid was collected and dried <u>in vacuo</u> to afford the <u>title ester</u> (2,365 g), m.p. 80.0 - 80.6° (Mettler), $\lambda_{max}$ ($CHCl_3$) 320 nm (ε 9,420).

<u>Example 12</u>

<u>(5RS)-2-(2-Aminoethyl)pen-2-em-3-carboxylic acid</u>

A solution of the product of Example 11 (1.0 g) in ethyl acetate-ethanol-water (1:1:1; 90 ml) containing 10% palladium on charcoal (6.0 g) was shaken and hydrogenated at room temperature and at 1 atmosphere pressure. After 15 minutes the mixture was filtered through kieselguhr and the pad was washed with a little water. The organic phase was separated, then extracted with water and the combined aqueous solutions were stirred and diluted with acetone. The resulting solid was collected and dried <u>in vacuo</u> to afford the <u>title</u>

acid (0.155 g), $\lambda_{max}$ (pH 6 buffer) 303 nm ($\varepsilon$ 4,840), $\nu_{max}$ (Nujol) 2700 br ($\overset{+}{N}H_3$), 1772 ($\beta$-lactam), 1562 $cm^{-1}$ ($CO_2^-$).

## Example 13

## 4-Azido-1-benzyloxycarbonyl-1-(2-oxo-4-triethyl ammonioazetidin-1-yl)but-1-en-2-olate

Triethylamine (3.37 ml) was added to a stirred solution of benzyl (3R,5R,Z)-2-(2-azidoethylidene) clavam-3-carboxylate (1.897 g) in ethyl acetate-ether (1:7, 40 ml). After 2 hours the resulting solid was collected, washed with ethyl acetate, ethyl acetate-ether ( 1:1), and ether (x3) , and then dried *in vacuo* to give the title salt (0.892 g), $\nu_{max}$ (Nujol) 2110 ($N_3$), 1784 ($\beta$-lactam), 1660 $cm^{-1}$ ($CO_2R$), $\tau$ (DMSO-$d_6$) values for mixture of isomers include 2.70 (s, aromatic protons), 4.38 + 4.88 (multiplets, azetidinyl C-4 H), 4.8 to 5.1 (M, bencylic protons), 8.84 +8.94 (triplets, J 7 Hz, $N(CH_2C\underline{H}_3)_3$).

## Example 14

## Benzyl (5RS)-2-(2-azidoethyl)pen-2-em-3-carboxylate

Methanesulphonyl chloride (0.48 ml) was added to a stirred solution of the product of Example 13 (0.857 g) in 1,2-dichloroethane (27 ml) containing pyridine (0.83 ml) and maintained at 0 to 5°. The solution was allowed to warm to room temperature and after 35 minutes was cooled to 5°. Hydrogen sulphide was passed through the reaction mixture under nitrogen and an excess of triethylamine was added in portions

over 10 minutes. The resulting mixture was diluted with 1,2-dichloroethane (30 ml) and, under nitrogen, was heated to boiling for 5 minutes. The solution was cooled to ambient temperature and washed successively with 0.5 N aqueous hydrochloric acid (twice), 0.5 N aqueous sodium hydrogen carbonate ( x 3), water, and saturated brine and then dried over magnesium sulphate . The filtered solution was evaporated to low volume, silica gel was added, and the resulting slurry was evaporated to dryness. The powder was transferred to the top of a silica gel column and eluted with ether-petroleum spirit (40 - 60°) (1:1). Appropriate fractions were combined and evaporated to yield the title ester (0.337 g) as an oil, $\lambda_{max}$ ($CHCl_3$) 318 nm (ε 7,300), $\nu_{max}$ ($CHBr_3$) 2104 ($N_3$), 1794 (β-lactam), 1710 $cm^{-1}$ ($CO_2R$).

Example 15

Benzyl (5RS)-2-(2-aminoethyl)pen-2-em-3-carboxylate

A solution of the product of Example 14 (0.100 g) in ethyl acetate-ethanol (1:1, 20 ml) containing 10% palladium on charcoal (0.3 g) was shaken and hydrogenated at room temperature and 1 atmosphere pressure. After 27 minutes the mixture was filtered through kieselguhr and the pad was washed with ethyl acetate-ethanol (1:1). The filtrate was evaporated to leave the title ester (0.070 g), $\nu_{max}$ ($(CH_2)_2Cl_2$) 1788 (β-lactam), and 1700 $cm^{-1}$ ($CO_2R$), τ (DMSO-$d_6$) values include 2.80 (s, aromatic protons), 4.46 (dd, J 4 and 2 Hz, C-5 H), 4.81 (s, benzylic protons).

Example 16

p-Nitrobenzyl (4'RS)-6-Azido-3-hydroxy-2-(2'-oxo-4'-p-tolylthioazetidin-1'-yl)hex-2-enoate

A solution of hexamethyldisilazane (6.72 ml), in dry tetrahydrofuran (136 ml) was cooled to -70° under nitrogen. n-Butyl lithium in hexane (1.52 molar solution, 21.1 ml) was added to the above solution and the temperature was allowed to fall over 15 minutes to -70°. A solution of p-nitrobenzyl (4'RS)-2-(2'-oxo-4'-p-tolylthioazetidin-1'-yl)acetate (7.08 g) in dry tetrahydrofuran (76 ml) was added dropwise over 4 minutes, the temperature was kept below -60°. The mixture was stirred for 3 minutes then 4-azido-butyryl chloride (2.4 ml, 20.15 mmole) was added dropwise. After a further 20 minutes at -70° the mixture was poured into 2N-hydrochloric acid (200 ml) and ethyl acetate (300 ml). The organic phase was separated, washed with saturated aqueous sodium bicarbonate solution and brine and dried (anhydrous sodium sulphate) and the solvent was removed in vacuo to give the crude title compound (9.44 g) as an oil with $\nu_{max}$ ($CHBr_3$) 2100 (azide), 1766 (β-lactam), 1662 (ester) and 1528 and 1350 $cm^{-1}$ ($NO_2$).

Example 17

p-Nitrobenzyl (2'RS)-6-Azido-3-methanesulphonyloxy-2-(2'-chloro-4'-oxoazetidin-1'yl)hex-2-enoate

The product of Example 16 (9.44 g) was dissolved in dry pyridine (24 ml) and methanesulphonyl chloride (2.78 ml) was added dropwise to the stirred mixture and

the temperature maintained at 0° to 5°. Water (4ml) was added dropwise to the above mixture and the product was stirred for 5 minutes and poured into a mixture of ethyl acetate and 2N-hydrochloric acid. The organic layer was separated and washed with brine. The aqueous phase was extracted with ethyl acetate which was washed with brine. The combined organic extracts were dried (sodium sulphate) and the solvent was evaporated in vacuo to give an oil (10.33 g). A cooled (0° to 5°) and stirred solution of this oil (10.33 g) in dry dichloromethane (108 ml) was treated with a solution of chlorine in carbon tetrachloride (27.2 ml of a 1.01 molar solution, equivalent to ca. 27.5 mmole of chlorine). After 1 hour the solution was evaporated in vacuo to give an oil which was dissolved in ethyl acetate and washed successively with a solution of sodium metabisulphite (17.4 g) in water (200 ml) and brine. The organic layer was dried (sodium sulphate) and evaporated in vacuo to give an oil (10.5 g). This oil was purified by chromatography on a column of Merck silica-gel (525 g) under a positive pressure of nitrogen (6 p.s.i.) and elution was with dichloromethane ethyl acetate (30:1). The appropriate fractions were collected and evaporated in vacuo to give the title compound (2.43 g) as a gum, $\lambda_{max}$ ($CHCl_3$) 261 nm ($E^{1\%}_{1cm}$ 316, ε 15,400), $\nu_{max}$ ($CHBr_3$) 2105 (azide), 1790 (β-lactam), 1734 ($CO_2R$), 1636 (C=C), 1528 and 1352 ($NO_2$) and 1375 $cm^{-1}$ ($RSO_2O$-).

Example 18

p-Nitrobenzyl (5RS)-2-Azidopropylpen-2-em-3-carboxylate

Hydrogen sulphide (3.36 ml of a 1.7 molar solution in N,N-dimethylformamide) was added to a stirred and cooled (-40°) solution of triethylamine (1.07 ml) in dry N,N-dimethylformamide (28 ml), under nitrogen. A solution of the product of Example 17 (1.85 g) in dry N,N-dimethylformamide (28 ml) was added over 15 minutes, the temperature was kept below -30°. Stirring was continued for a further hour at -38°. The mixture was poured into ethyl acetate-brine and the organic phase was separated and washed with brine (6 times) then dried (sodium sulphate) and the solvent was evaporated in vacuo to give the title compound (2.1 g) as a brown gum, $\lambda_{max}$ ($CHCl_3$) 266.5 nm ($E^{1\%}_{1cm}$ 322, ε 12,500) with an inflection at 310 mn ($E^{1\%}_{1cm}$ 172, ε 5,700), $\nu_{max}$ ($CHBr_3$) 2110 (azide), 1794 (β-lactam), 1714 ($CO_2R$), 1582 (C=C), 1524 and 1350 $cm^{-1}$ ($NO_2$).

Example 19

(5RS)-2-[3-Aminopropyl]-pen-2-em-3-carboxylate

10% Palladium on charcoal (1.9 g) was added to a stirred solution of the product of Example 18 (2.1 g) in ethyl acetate (100 ml). After 3 minutes the mixture was filtered through kieselguhr and the filtrate was evaporated in vacuo to give a gum. 10% Palladium on charcoal (10.5 g) was added to a solution of the gum in a mixture of ethyl acetate (68 ml), ethanol (68 ml). The resultant suspension was hydrogenated at 21° and atmospheric pressures for 15 minutes then filtered

through kieselguhr and the pad was washed with water. The filtrate was extracted with ethyl acetate and ether. The solvent residues were evaporated in vacuo to give a glass (0.322 g). This product was purified by passage through a column of XAD-2 resin (50 g) eluting with ethanol:water (1:3).. Appropriate fractions were collected and were evaporated in vacuo and freeze-dried and dried over phosphorus pentoxide to give two fractions (0.178 g and 0.099 g respectively) as powders. The second crop consisted of pure title compound with $\lambda_{max}$ ($H_2O$) 254.5 nm ($E^{1\%}_{1cm}$ 151, ε 3,400) and 300.5 nm ($E^{1\%}_{1cm}$ 188, ε 4,200), $\nu_{max}$ (Nujol) 3400 ($H_2O$), 2630 ($NH_3^+$), 1772 (α-lactam), 1590 ($CO_2^-$) and 1568 $cm^{-1}$ (C=C).

The following Examples illustrate how compounds according to the invention maybe formulated into pharmaceutical compositions.

Example A

Formula per tablet

| | |
|---|---|
| Densified (5RS)-2-(2-aminoethyl)pen-2-em-3-carboxylic acid containing 1% magnesium stearate | 302.0 mg |
| Empicol LZ | 4.5 mg |
| Explotab | 9.0 mg |
| Avicel PH 101 to tablet core weight of | 450.0 mg |

Method of Preparation

Blend the antibiotic with magnesium stearate and compress into slugs on a heavy duty tableting machine. Break down the slugs through a series of screens (10,12, 16 and 20 mesh) on a rotary granulator to produce free-flowing granules. Blend the granules with the rest of the excipients and compress the blend on a tablet machine using normal or deep concave punches (9 - 12 mm diameter).

Film coat the tablet cores using hydroxypropylmethyl cellulose or similar film forming material using either an aqueous or non-aqueous solvent system. A plasticizer and suitable colour may be included in the film coating solution.

Example B

Dry Powder for Injection

Fill sterile 5RS-2-(2-aminoethyl)pen-2-em-3-carboxylic acid aseptically into glass vials under a blanket of sterile nitrogen using a fill-weight of 500 mg per vial. Close the vials using rubber discs or plugs held in position by aluminium sealing rings, thereby preventing gaseous exchange or ingress of microorganisms. Constitute the product by dissolving in Water for Injections shortly before administration.

Empicol LZ is sodium lauryl sulphate available from Marchon Ltd.; Explotab is sodium starch glycolate available from Greeff Fine Chemicals Ltd., Croydon, Surrey, England; Avicel PH 101 is microcrystalline cellulose available from FMC Corporation, U.S.A.

Results of Biological Tests

Biological testing of two of the compounds of the invention has provided the results shown below; the test compounds are identified by their Example Numbers:

1. Determination of Minimum Inhibitory Concentrations (MIC)

Serial two-fold dilutions of freshly prepared test solutions were made into Oxoid No.1 Nutrient agar with or without added enrichment and poured into petri diches Plates were inoculated with a multipoint inoculator with inoculum containing approximately $10^5$ colony forming units of the organisms shown in the following Tables, all of which organisms are clinical isolates. The MIC, quoted in the Table as µg/ml, was read after 18 hours incubation at 37°C as the lowest concentration which inhibited growth.

| Organism | | Example No: 4 | 19 |
|---|---|---|---|
| Staph. aureus | 853E | 1 | 0.5 |
| Micrococcus Sp | 1810E | 0.2 | 0.1 |
| E. coli | 851E | 8 | 8 |
| E. cloacae | 1051E | 8 | 16 |
| E. cloacae | 1321E | 8 | 8 |
| K. aerogenes | 1522E | 16 | 16 |
| S. marcescens | 1324E | 31 | 31 |
| H. influenzae | 1184E | 62 | 16 |

Claims

1. A process for the preparation of compounds of the formula (I)

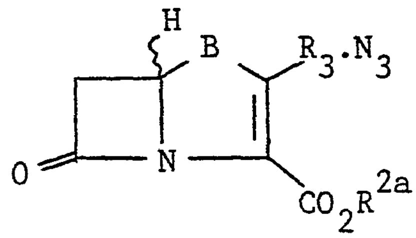

(I)

wherein $R^{2a}$ represents a carboxyl esterifying group or a hydrogen atom, $R^3$ represents a group $-C_nH_{2n}-$ where n is an integer from 1 to 6, and B represents $>S$ or $>S \rightarrow O$ together with salts thereof,

in which a compound of formula (II)

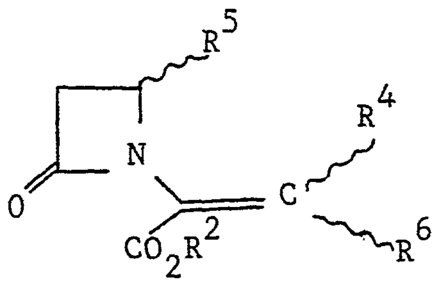

(II)

wherein $R^2$ represents a carboxyl esterifying group as defined above for $R^{2a}$; $R^4$ represents a group of formula $-R^3N_3$ where $R^3$ is as defined above;

$R^5$ represents a halogen atom or a group of formula $-\overset{\oplus}{N}R^xR^yR^zA^{\ominus}$ (in which $R^x$, $R^y$ and $R^z$, which may be the same or different, each represents an aliphatic, araliphatic or aromatic group or two of $R^x$, $R^y$ and

$R^z$ together with the nitrogen atom to which they are attached represent a five-, six- or seven-membered heterocyclic ring optionally containing a further heteroatom and the third of $R^x$, $R^y$ and $R^z$ is as defined above or $R^x$, $R^y$ and $R^z$ together with the nitrogen atom to which they are attached form an aromatic heterocyclic ring or an aromatic or non-aromatic polycyclic heterocyclic system and $A^{\ominus}$ is an anion); and

$R^6$ represents a leaving group, is reacted with hydrogen sulphide or with a hydrosulphide or sulphide salt, followed, where a free acid or salt is desired, by deesterification and salt formation and/or where a compound of formula (I) in which B is $>S\rightarrow O$ is desired, by oxidation.

2. A process as claimed in claim 1 wherein n is 2 and B is $>S$.

3. A process as claimed in claim 1 or claim 2 wherein cyclisation is effected using hydrogen sulphide in the presence of a base.

4. A process as claimed in any of claims 1 to 3 wherein a compound of formula (II) in which $R^5$ represents a chlorine or bromine atom or a group $-\overset{\oplus}{N}(C_2H_5)_3\ A^{\ominus}$ is used, wherein $A^{\ominus}$ is an anion.

5. A process as claimed in any one of claims 1-4 wherein a compound of formula (II) in which $R^6$ represents the residue of an acid $R^6H$ having a $pk_a$ (in water at 25°C) of 3.5 or less.

6. Compounds of the formula (I) as defined in claim 1.

7. Compounds as claimed in claim 6 wherein B represents a sulphur atom >S.

8. Compounds as claimed in claim 6 or claim 7 wherein n in the group $R^3$ is from 1 to 4.

9. Compounds as claimed in any of claims 6-8 wherein $R^{2a}$ represents a straight or branched substituted or unsubstituted alkyl or alkenyl group having from 1-8 carbon atoms; an aralkyl group having up to 20 carbon atoms; an aryl group having up to 12 carbon atoms; or a cycloalkyl group having up to 12 carbon atoms; a heterocyclic group having up to 12 carbon atoms, or a silyl or stannyl group having up to 24 carbon atoms.

10. Compounds as claimed in any of claims 6-9 wherein n in the group $R^3$ is 1, 2 or 3 and $R^{2a}$ is a benzyl or 4-nitrobenzyl group.

11. Compounds as claimed in claim 10 wherein n is 2.

12.. Compounds of the formula (I)

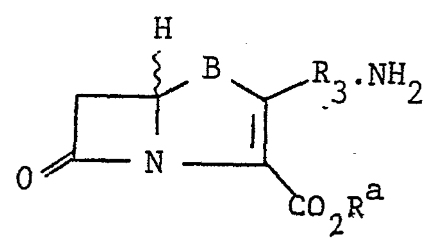

(I)

wherein $R^{2a}$ represents a carboxyl esterifying group or a hydrogen atom, $R^3$ represents a group $-C_nH_{2n}-$ where n is an integer from 1 to 6, and B represents a group $>S$ or $>S\rightarrow O$, together with salts and zwitterionic forms thereof.

13. A process for the preparation of compounds of formula (I) as defined in claim 12 which comprises reducing a compound of formula (I) as claimed in claim 6.

14. Pharmaceutical compositions comprising as active ingredient a compound of formula (I) as defined in claim 6 or claim 12 in which $R^{2a}$ represents a hydrogen atom or a metabolically labile carboxyl esterifying group or a salt of such a compound of formula (I) in association with a pharmaceutical carrier or excipient.

15. Compounds of the general formula (XIII),

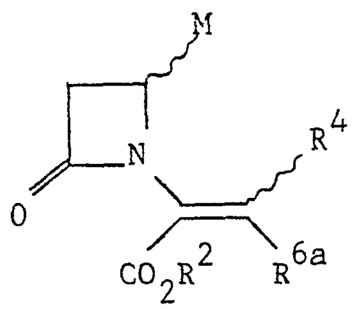

(XIII)

wherein $R^4$ is as defined in claim 1 ; $R^2$ represents a carboxyl esterifying group; and either M represents a halogen atom or a group $R^9$ (in which $R^9$ represents a group $-SR^{10}$ or

$-\overset{O}{\overset{\uparrow}{S}}-R^{10}$ where $R^{10}$ is an aliphatic, araliphatic,

aromatic or heterocyclic group) and $R^{6a}$ represents a hydroxy group or a leaving group;
or M represents a group of formula

$$-\overset{\oplus}{N}R^xR^yR^zA^{\ominus}$$

(where $R^x$, $R^y$, $R^z$ and $A^{\ominus}$ are as defined in claim 1) and $R^{6a}$ represents a leaving group;
or M represents a group $\overset{+}{N}R^xR^yR^z$ (where $R^x$, $R^y$ and $R^z$ are as hereinbefore defined) and $R^{6a}$ represents $S^{\ominus}$ or $O^{\ominus}$.

16. Compounds of the formula

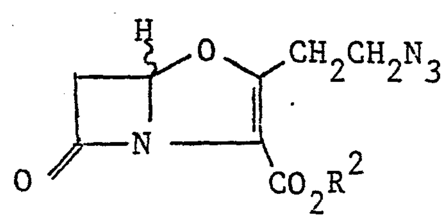

(V)

wherein $R^2$ is as defined in claim 1.